# EUROPÄISCHE PATENTSCHRIFT

(11) **EP 1 644 941 B1**
(45) Veröffentlichungstag und Bekanntmachung des Hinweises auf die Patenterteilung: **23.11.2011**
(21) Anmeldenummer: 04740828.1
(22) Anmeldetag: 09.07.2004
(51) Int. Cl.: H01F 1/00, H01F 1/42, H01F 1/44

(54) **VERFAHREN ZUR HERSTELLUNG MAGNETISCHER NANOPARTIKEL MIT VERBESSERTEN MAGNETEIGENSCHAFTEN**
METHOD OF MANUFACTURING MAGNETIC NANOPARTICLES HAVING IMPROVED MAGNETIC PROPERTIES
PROCEDE DE FABRICATION DES NANOPARTICULES MAGNETIQUES AUX PROPRIETES MAGNETIQUES AMELIOREES

(30) Priorität: 10.07.2003 DE 10331439
(43) Veröffentlichungstag der Anmeldung: 12.04.2006
(73) Patentinhaber: micromod Partikeltechnologie GmbH, 18119 Rostock-Warnemünde (DE)
(72) Erfinder: TELLER, Joachim, 18276 Mistorf (DE); WESTPHAL, Fritz, 18184 Poppendorf (DE); GRÜTTNER, Cordula, 18273 Güstrow (DE)
(74) Vertreter: Garrels, Sabine
(86) Internationale Anmeldenummer: PCT/EP2004/007539
(87) Internationale Veröffentlichungsnummer: WO 2005/006356

(56) Entgegenhaltungen:
- EP-A- 0 699 964
- WO-A-95/27437
- US-A- 4 501 726
- US-A- 5 667 716
- US-A- 5 814 687
- US-A- 5 852 076

## Beschreibung

Die Erfindung betrifft ein Verfahren zur Herstellung magnetischer Nanopartikel, die aus Metalloxid-Polymerkompositen bestehen.

### [Stand der Technik]

Für magnetische Kompositpartikel mit Durchmessern im Nanometerbereich gibt es bereits eine Reihe von technisch etablierten Applikationen. Beispielsweise können derartige Partikel in molekularbiologischen Applikationen zur Isolierung, Fixierung und Reinigung von Zellen, Zellbestandteilen, Nukleinsäuren, Enzymen, Antikörpern, Proteinen und Peptiden, in der Zellbiologie zu Phagozytose-Untersuchungen, in der Klinischen Chemie als Bestandteil von diagnostischen Assays oder therapeutischen Arzneiformen, in der Klinischen Diagnostik als Kontrastmittel, Radionuklid- oder Drug-Carrier in der Biochemie und Technischen Chemie als Festphasen für die Untersuchung von Molecular Recognition-Phänomenen und heterogenkatalytischen Prozessen eingesetzt werden.

Eine Vielzahl von polymerbeschichteten Metalloxidpartikeln wurden seit Mitte der 1980-er Jahre für biologische Anwendungen in magnetischen Feldern beschrieben. Insbesondere magnetisierbare Nanopartikel unterhalb von 200 nm eröffnen neue Möglichkeiten für den Transport und die Separation von Zellen, Zellbestandteilen, bioaktiven Molekülen und Radionukliden (US2003/0099954 MILTENYI; WO01/17662 ZBOROWSKI; WO02/43708 ALEXIOU), für die Anwendung als Markierung in kontrastgebenden magnetischen Abbildungs- und Diagnoseverfahren (US2003/0092029A1 JOSEPHSON; WO01/74245 JOHANSSON; US5427767 KRESSE) sowie die mechanische (DE10020376A1 KOCH) und thermale Beeinflussung von lebenden Zellen (US6541039 LESNIAK) und sind deshalb ständig in ihren applikationsbezogenen Eigenschaften verbessert worden. Allen Anwendungen ist gemeinsam, dass magnetisierbare Metalloxide mit einer biokompatiblen polymeren Beschichtung zu Kompositpartikeln mit Größen von 5 nm bis 500 nm zu einer kolloidal stabilen Suspension auf wässriger Basis verbunden werden. Dabei soll das Beschichtungmaterial entweder eine Wechselwirkung mit biologischen Materialien ausschließen, eine gute Verträglichkeit mit lebenden Zellen ermöglichen und die Wege der Metabolisierung in lebenden Organismen beeinflussen oder durch gezielte Funktionaliserung mit biochemisch aktiven Substanzen eine selektive Bindung an der Oberfläche ermöglichen oder kontrolliert eingeschlossene Substanzen freisetzen. Mittels der magnetisierbaren Anteile der Kompositpartikel wird dabei eine energetische Wechselwirkung mit äußeren magnetischen Feldern genutzt. In magnetischen Feldern erfahren solche Partikel abhängig von den magnetischen Eigenschaften eine Ausrichtung, sie bewegen sich entsprechend räumlicher magnetischer Feldgradienten und reagieren auf zeitliche Änderungen des äußeren Magnetfeldes.

Eine große Anzahl von Methoden wurde zur Herstellung von Eisenoxidkristalliten als Metalloxidpartikel beschrieben etwa durch Sinterung bei hohen Temperaturen mit anschließender mechanischer Zerkleinerung, Clusterbildung unter Vakuumbedingungen oder nasschemische Synthese aus Lösungen. Die Fällung von Eisenoxiden kann unter nicht-wässrigen Bedingungen erfolgen (US4677027 PORATH) und anschließend in wässrige Bedingungen überführt werden (US5160725 PILGRIM) oder ausschließlich in wässrigen Lösungen erfolgen (US4329241 MASSART). Aus toxikologischen Gründen wird für biologische Anwendungen eine wässrige Formulierung verwendet (US4101435 HASEGAWA). Die nasschemische Synthese der Eisenoxidkristallite kann einer Beschichtung durch die polymeren Bestandteile vorangehen (Core-shell-Methode) oder in Anwesenheit des Polymers erfolgen (One-pot-Methode). Die Core-shell-Methode macht die Zugabe von Stabilisatoren zu den Eisenoxiden erforderlich, da diese zur Bildung von Aggregaten in wässriger Suspension neigen. Als Stabilisatoren kommen amphiphile Substanzen (WO01/56546 BABINCOVA) oder zusätzliche Nanopartikel mit elektrisch geladener Oberfläche in Betracht (US4280918 HOMOLA). Oberflächenaktive Substanzen als Stabilisatoren können jedoch die Möglichkeiten einer chemischen Funktionalisierung der Oberfläche stark eingrenzen. Heute werden im allgemeinen hauptsächlich nach der One-pot-Methode hergestellte magnetisierbare eisenhaltige Nanokompositpartikel wegen ihrer physikalischen und chemischen Eigenschaften und der pharmazeutischen/galenischen Stabilität für medizinische Anwendungen akzeptiert.

Die One-pot-Methode verwendet das Beschichtungspolymer direkt während der Bildung der Eisenoxide zur Stabilisierung bei Keimbildung und Wachstum der Kristallite aus der Lösung. Eine der meist eingesetzten Beschichtungsmaterialien ist Dextran in verschiedenen Modifizierungen. Aber auch andere Polysaccharide wie Arabinogalactan, Stärke, Glycosaminoglycane oder Proteine fanden Verwendung (US6576221 KRESSE). Das wohl einfachste Verfahren ist die Fällung von Eisen(II)- und Eisen(III)-Salzen in Gegenwart von Dextran (US4452773 MOLDAY). Abgewandelt wird das Verfahren durch die Verwendung von Ultraschall mit anschließender thermischer Behandlung in einem Durchflussverfahren (US4827945 GROMAN). Die Qualität des Produktes kann durch magnetische Klassierung weiter verbessert werden (W09007380 MILTENYI). Eine weitergehende Verkapselung/Beschichtung meist unter Verwendung amphiphiler Substanzen als Stabilisatoren kann das Verhalten biologischer Systeme gegenüber den Kompositpartikeln wesentlich modifizieren (US5545395 TOURNIER, EP0272091 ELEY).

Zur Herstellung hoch disperser wässriger Systeme als injizierbare Flüssigkeit kommen neben verschiedenen Stabilisatoren unterstützend spezielle Methoden der Homogenisierung zum Einsatz. Solche Methoden sind beispielsweise die Rotor-Stator-Homogenisierung und die Hochdruckhomogenisierung. Ein besonders hoher mechanischer Energieeintrag wird durch den Einsatz von Liquidjet- oder Liquid-slot-nozzle-Hochdruckhomogenisatoren erreicht (Microfluidizer-Technologie), was insbesondere zur Herstellung von Liposomen genutzt wird (US5635206 GANTER) aber auch in anderen Fällen die Herstellung injizierbarer Wirkstoffformulierungen erleichtert (US5595687 RAYNOLDS). Der Einsatz eines Hochdruckhomogenisators zur Herstellung oxidischer Nanokompositpartikel mittels kontrollierter Koaleszenz mit anschließender Trocknung in Emulsionen, deren nichtwässrige Komponente eine Oxidkomponente als Sol enthält, ist im Zusammenhang mit der industriellen Herstellung von Katalysatormaterialien (US5304364 COSTA) sowie elektrographischer Tonerpartikel, keramischer Pulver, Filzmaterialien, Sprühschichten, Wirkstoffträger oder lonenaustauscherharze beschrieben (US5580692 LOFFTUS).

In "Magnetische Flüssigkeiten - Ferrofluide" (A.Nethe, Lehrstuhl Theoretische Elektrotechnik und Prozessmodelle, Brandenburgische Universität Cottbus, 2001) werden magnetische Nanopartikel offenbart, welche aus Metalloxiden und einem Polymer bestehen, ca. 50 Masseprozent Metall enthalten und einen hydrodynamischen Durchmesser unterhalb von 200nm aufweisen. Es handelt sich hierbei um ein typisches Verfahren, um die sogenannten SPIO-Partikel zu erzeugen.

Alle beschriebenen Magnetpartikel-Typen im Größenbereich unterhalb 200 nm sind meist nur über aufwendige Separationsverfahren (z. B. Hochgradienten-Magnet-separation) anzureichern oder zu fixieren.

Andererseits gibt es in den Life Sciences bereits zahlreiche Magnetpartikelapplikationen, die durch Separationsschritte an Permanentmagneten wesentlich effizienter ausgeführt werden könnten oder die aus anderen Gründen eine hohe Magnetomobilität der Partikel erfordern.

### [Aufgabe der Erfindung]

Somit lag der vorliegenden Erfindung die Aufgabe zugrunde, magnetische Nanopartikel zugänglich zu machen, die über eine genügend hohe Magnetisierung bei kleinen Feldstärken verfügen.

Diese Aufgabe wird dadurch gelöst, dass magnetische Nanopartikel bestehend aus einem Metalloxid und einem Polymer mit einem Massenanteil an Metall größer oder gleich 50 % und hydrodynamischen Durchmessern unterhalb 200 nm mittels Hochdruckhomogenisierung aus den Komponenten und einem Trägermedium erzeugt werden.

Diese magnetischen Nanopartikel sind weiterhin dadurch gekennzeichnet, dass sie bei kleinen Magnetfeldstärken ein vergleichsweise höheres magnetisches Moment als das eingesetzte Metalloxid aufweisen.

Solche magnetische Nanopartikel werden weder durch Amphiphile strukturiert, wie es bei Magnetliposomen der Fall ist, oder durch Tenside stabilisiert, so wie üblicherweise bei Ferrofluiden verfahren wird. Vielmehr bilden sie in Wasser und wässrigen Lösungen ohne Einwirkung eines äußeren Magnetfeldes ein langzeitstabiles Kolloid.

Bei den eingesetzten Metalloxiden handelt es sich vornehmlich um Eisenoxide, wie Magnetit (Fe₃O₄) oder Maghemit (Fe₂O₃) oder daraus resultierenden Mischphasen. Die Eisenoxide können durchaus auch Anteile von anderen zwei-oder dreiwertigen Metallionen, wie beispielsweise Ca²⁺, Ba²⁺, Zn²⁺, Co²⁺, Co³⁺, Cr³⁺, Ti³⁺, Mo²⁺, Mn²⁺ und Cu²⁺, enthalten.

Das eingesetzte Polymer kann dem Bereich der synthetischen Polymere entstammen. Dafür kommen prinzipiell Polymere in Frage, die über Heteroatome oder funktionelle Gruppen verfügen, die bindende Wechselwirkungen zu Metallionen eingehen können, wie unter anderem Polyole, Polyamine, Polyether, Polyester, Polyamide sowie davon abgeleitete Derivate, Copolymere und Blends.

Andererseits kann das Polymer auch aus der Gruppe der Biopolymere und hier insbesondere aus dem Bereich der Polysaccharide gewählt werden. Dabei kommen sowohl natürliche als auch derivatisierte Polysaccharide in Betracht. Unter den Polysacchariden weisen eine Reihe von Vertretern eine ausgeprägte Affinität zu Schwermetallionen, insbesondere auch Eisenionen, auf.

Zu diesen Vertretern zählt auch das Dextran, das zudem den Vorteil bietet, weniger als andere natürliche Polysaccharide (z. B. Stärke) Qualitätsschwankungen zu unterliegen, was wiederum für die Reproduzierbarkeit der Partikelchargen von großem Wert ist. Ebenso ist eine Derivatisierung des Dextrans in vielfältiger Weise ausführbar. Nach an sich bekannten Methode können so funktionelle Gruppen (COOH, NH₂, ...), Spacer mit funktionellen Gruppen (Polyethylenglykol-basierte COOH- oder NH₂-Gruppen) oder biochemisch relevante Substrukturen (Oligonukleotide, Nukleinsäuren, Peptide, Proteine sowie Antikörper und Enzyme) eingeführt werden. Die Derivatisierung des Dextrans kann aber auch genutzt werden, um metallselektive Chelatoren, beispielsweise für die Fixierung von Radionukliden, oder pharmazeutischen Wirkstoffen zu binden.

Als Technologie zur Herstellung der erfindungsgemäßen magnetischen Nanopartikel hat sich die Hochdruckhomogenisation unter Verwendung des Microfluidizer™ vom Typ M-110Y bewährt. Dabei werden die Komponenten Metalloxid und Polymer in einem Trägermedium, in den meisten Fällen wird Wasser verwendet, bei Drücken im Bereich von 500 bar bis 1200 bar unter Anwendung hoher Scherkräfte prozessiert. Das Verfahren kann auch dadurch modifiziert werden, dass die Metalloxide erst während der Ultrahomogenisation aus entsprechenden Metallsalzen oder-hydroxiden in situ generiert werden. In diesen Fällen wird ein alkalisches Trägermedium, beispielsweise eine wässrige Ammoniaklösung verwendet.

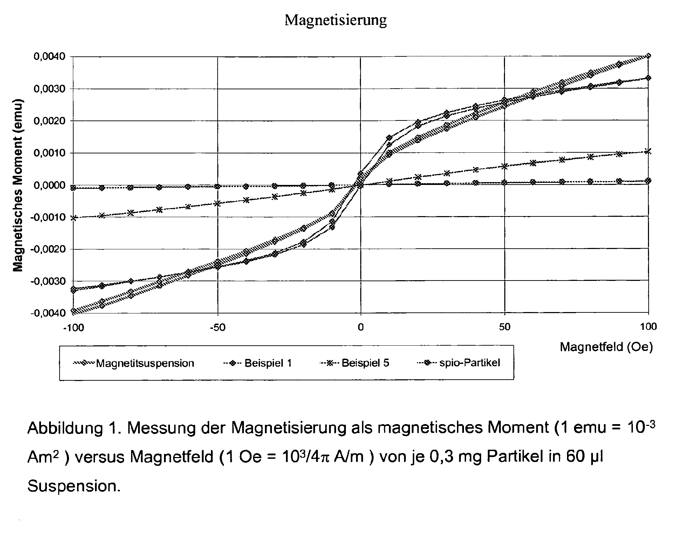

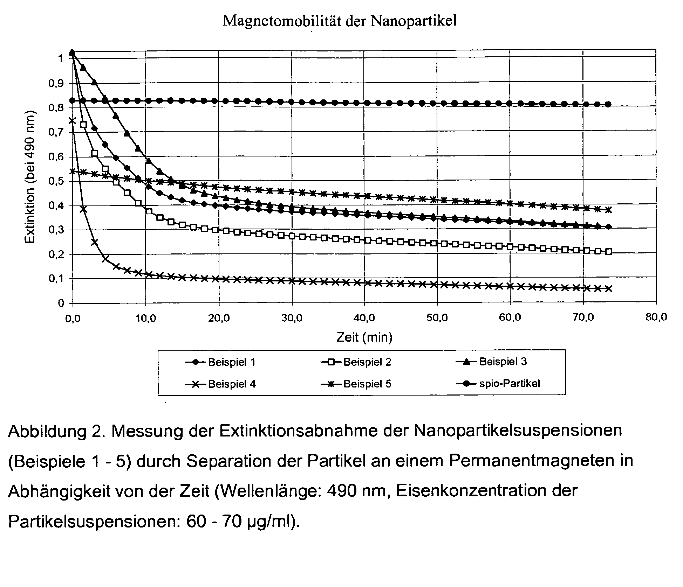

Überraschenderweise konnte festgestellt werden, dass die Hochdruckhomogenisierung der Komponenten Metalloxid und Polymer in einem Trägermedium nicht nur zu kolloidal stabilen Magnetpartikel-Population im Durchmesserbereich unterhalb von 200 nm führt, sondern dass die erzeugten magnetischen Nanopartikel bei kleinen Magnetfeldstärken unter 50 Oe in Suspension über größere magnetische Momente verfügen als sie für das als Ausgangsmaterial verwendete Metalloxid bestimmt wurden (Abb. 1).Die Auswirkung der verbesserten Magneteigenschaften auf die Magnetomobilität wird beim Vergleich der ermittelten Werte für die erfindungsgemäß hergestellten magnetischen Nanopartikeln mit herkömmlichen superparamagnetischen Eisenoxid-Partikeln (SPIO) deutlich (Abb. 2). Die nach den Beispielen 1 bis 5 gewonnenen magnetischen Nanopartikel weisen durchweg wesentlich höhere Magnetomobilitäten als vergleichbare SPIO-Partikel ( analog US 4452773, Partikeldurchmesser: 100 nm) auf.

Die erfindungsgemäß hergestellten magnetischen Nanopartikel können für diverse Life-Sciences-Applikationen eingesetzt werden. So sind sie besonders für Anwendungen auf bioanalytischem und diagnostischem Gebiet, bei Bioseparationsprozessen und als Trägermaterial im High-Throughput-Screening geeignet. Der geringe Durchmesser in Verbindung mit der ausgeprägten kolloidalen Stabilität erlaubt darüber hinaus auch ihren Einsatz bei in vivo-Anwendungen, beispielsweise in Form von injezierbaren Kontrastmitteln, Radionuklid-Carriern oder Wirkstoffdepots. Für solche Applikationen ist es von besonderem Vorteil, dass die erfindungsgemäßen Partikel durch Sterilfiltration aufbereitet werden können.

### [Beispiele]

Die Erfindung soll anhand der nachfolgenden Beispiele näher erläutert werden, ohne darauf beschränkt zu sein.

### Beispiel 1.

36 g Dextran (MW = 40.000 D, Fluka) wurden in 120 ml Wasser gelöst. 180 ml einer 2,5% (w/w) wässrigen Magnetitsuspension (micromod, 45-00-202, Partikeldurchmesser: 200 nm) wurde auf 40°C erwärmt und 10 min bei 500 bar im Microfluidizer M-110Y ultrahomogenisiert. Nach Druckerhöhung auf 1000 bar wurde die auf 40°C erwärmte Dextranlösung zur ultrahomogenisierten Magnetitsuspension gegeben. Die Dextran-Magnetit-Suspension wurde 20 min bei 1000 bar und 90°C ultrahomogenisiert. Nach Abkühlen auf Raumtemperatur wurden die erhaltenen magnetischen Nanopartikel zur Abtrennung des Dextranüberschusses 15 min am Permanentmagneten separiert und in 40 ml Wasser resuspendiert. Der hydrodynamische Durchmesser der resultierenden magnetischen Nanopartikel beträgt 130-140 nm (Photonenkorrelationsspektroskopie, Zetasizer 3000, Malvern Instr.). Der Eisenanteil in den Partikeln beträgt 58-62 % (w/w).

### Beispiel 2.

Die Partikelsynthese wurde analog Beispiel 1. durchgeführt, wobei der Druck während der gesamten Ultrahomogenisation 500 bar betrug. Der hydrodynamische Durchmesser der resultierenden magnetischen Nanopartikel beträgt 160-180 nm (Photonenkorrelationsspektroskopie, Zetasizer 3000, Malvern Instr.). Der Eisenanteil in den Partikeln beträgt 58-62 % (w/w).

### Beispiel 3.

Die Partikelsynthese wurde analog Beispiel 1. durchgeführt, wobei das Massenverhältnis zwischen Dextran und Magnetit von 8:1 (Beispiel 1) auf 12:1 erhöht wurde. Dazu wurden 54 g Dextran (MW = 40.000 D, Fluka) in 180 ml Wasser gelöst. Der hydrodynamische Durchmesser der resultierenden magnetischen Nanopartikel beträgt 130-140 nm (Photonenkorrelationsspektroskopie, Zetasizer 3000, Malvern Instr.). Der Eisenanteil in den Partikeln beträgt 52-56 % (w/w).

### Beispiel 4.

12 g Ethylenimin-Polymerlösung (50% (v/v), MW = 600 - 1.000 kD, Fluka) wurde mit 30 ml Wasser gemischt. 60 ml einer 2,5% (w/w) wässrigen Magnetitsuspension (micromod, 45-00-202, Partikeldurchmesser: 200 nm) wurde auf 40°C erwärmt und 10 min bei 500 bar im Microfluidizer M-110Y ultrahomogenisiert. Nach Druckerhöhung auf 1000 bar wurde die auf 40°C erwärmte Ethylenimin-Polymerlösung zur ultrahomogenisierten Magnetitsuspension gegeben. Die Polyethylenimin-Magnetit-Suspension wurde 20 min bei 1000 bar und 90°C ultrahomogenisiert. Nach Abkühlen auf Raumtemperatur wurden die erhaltenen magnetischen Nanopartikel zur Abtrennung des Polymerüberschusses 15 min am Permanentmagneten separiert und in 25 ml Wasser resuspendiert. Der hydrodynamische Durchmesser der resultierenden magnetischen Nanopartikel beträgt 80 nm (Photonenkorrelationsspektroskopie, Zetasizer 3000, Malvern Instr.). Der Eisenanteil in den Partikeln beträgt 60-65 % (w/w).

### Beispiel 5.

72 g Dextran (MW = 40.000 D, Fluka) wurden in 180 ml Wasser gelöst. 90 ml einer 1,5% (w/w) wässrigen Maghemitsuspension, Partikeldurchmesser: 20 nm, pH= 1.6 - 2.0) wurde auf 40°C erwärmt und 5 min bei 500 bar im Microfluidizer M-110Y ultrahomogenisiert. Nach Zugabe der auf 40°C erwärmten Dextranlösung zur ultrahomogenisierten Maghemitsuspension wurde die Suspension durch Zugabe von 120 ml 0.1 M Natronlauge neutralisiert. Nach Abkühlen auf Raumtemperatur wurden die erhaltenen magnetischen Nanopartikel mittels Hochgradientenmagnetfeld mit Wasser gewaschen. Der hydrodynamische Durchmesser der resultierenden magnetischen Nanopartikel beträgt 60-70 nm (Photonenkorrelationsspektroskopie, Zetasizer 3000, Malvern Instr.). Der Eisenanteil in den Partikeln beträgt 50-52 % (w/w).

### Beispiel 6.

Zur Funktionalisierung mit terminalen Carbonsäuregruppen über einen Ethylenglycol-Spacer wurden 20 ml einer 5 % (w/w) Dextran-Magnetit-Nanopartikel-Suspension aus Beispiel 1 (Partikelduchmesser: 130-140 nm) mit 5 ml 0,5 M 2-Morpholino-ethansulfonsäure-Puffer (pH = 6,3) gemischt. 120 mg N-Ethyl-N'-(3-dimethylaminopropyl)-carbodiimid-hydrochlorid und 120 mg 3,6-Dioxaoctandisäure wurden in je 5 ml 0,1 M 2-Morpholino-ethansulfonsäure-Puffer (pH = 6,3) gelöst und vereinigt. Nach Inkubation dieser Lösung für 10 min bei 50°C erfolgte die Zugabe zur Nanopartikel-Suspension. Die Partikelsuspension wurde 2 h bei Raumtemperatur geschüttelt. Nach Separation am Permanentmagneten wurden die Nanopartikel in Wasser resuspendiert. Für die Dichte an Carbonsäuregruppen auf der Partikeloberfläche wurde ein Wert von 40-50 nmol/mg mittels Strömungspotenzialmessung bestimmt (Polyelektrolyttitration gegen 0,001 N Poly(diallyldimethylammoniumchlorid)-Lösung, Mütek PCD 03 pH). Der hydrodynamische Durchmesser der resultierenden magnetischen Nanopartikel beträgt 120-130 nm (Photonenkorrelationsspektroskopie, Zetasizer 3000, Malvern Instr.). Der Eisenanteil in den Partikeln beträgt 60-65 % (w/w).

### Beispiel 7.

Zur kovalenten Bindung von Streptavidin auf der Partikeloberfläche wurden 10 ml einer 2 % (w/w) Dextran-Magnetit-Nanopartikel-Suspension aus Beispiel 6 mit terminalen Carbonsäuregruppen auf der Partikeloberfläche mit 2,5 ml 0,5 M 2-Morpholino-ethansulfonsäure-Puffer (pH = 6,3) gemischt. 20 mg N-Ethyl-N'-(3-dimethylaminopropyl)-carbodiimid-hydrochlorid und 40 mg N-Hydroxysuccinimid wurden in je 1 ml 0,1 M 2-Morpholino-ethansulfonsäure-Puffer (pH = 6,3) gelöst und zur Nanopartikel-Suspension gegeben. Die Partikelsuspension wurde 2 h bei Raumtemperatur geschüttelt. Nach Separation am Permanentmagneten wurden die Nanopartikel in 10 ml 0,1 M 2-Morpholino-ethansulfonsäure-Puffer (pH = 6,3) resuspendiert. Nach Zugabe von 1 mg Streptavidin (Molecular Probes) wurde die partikelsuspension 3 h bei Raumtemperatur geschüttelt. Zur Absättigung reaktiver Stellen wurde die Partikel-Suspension nach Zugabe von 2 ml 0,4 M Glycinlösung eine weitere Stunde bei Raumtemperatur geschüttelt. Nach Separation am Permanentmagneten wurden die Nanopartikel einmal mit 10 ml PBS-Puffer (pH=7,4) gewaschen und in 5 ml PBS-Puffer (pH=7,4) resuspendiert. Die Konzentration an kovalent gebundenem Streptavidin auf den Dextran-Magnetit-Nanopartikeln beträgt 1,5-2 µg Streptavidin pro mg Partikel. Der hydrodynamische Durchmesser der resultierenden magnetischen Nanopartikel beträgt 130-140 nm (Photonenkorrelationsspektroskopie, Zetasizer 3000, Malvern Instr.). Der Eisenanteil in den Partikeln beträgt 60-65 % (w/w).

## Patentansprüche

1. Verfahren zur Herstellung magnetischer Nanopartikel mit den Eigenschaften, dass sie a) 50 oder mehr Masseprozent Metall enthalten, b) hydrodynamische Durchmesser unterhalb von 200 nm aufweisen und c) bei Magnetfeldstärken unter 50 Oe in Suspension eine höhere Magnetisierung als das eingesetzte Metalloxid aufweisen, **gekennzeichnet dadurch, dass**
- eine wässrige Eisenoxidsuspension erwärmt und bei mindestens 500 bar ultrahomogenisiert wird,
- ein in Wasser gelöstes Polymer erwärmt und zur ultrahomogenisierten Eisenoxidsuspension gegeben wird,
- die Polymer-Eisenoxid-Suspension unter weiterer Erwärmung und einem Druck von 500 bar oder darüber ultrahomogenisiert wird und
- nach Abkühlen auf Raumtemperatur die erhaltenen magnetischen Nanopartikel zur Abtrennung des Polymerüberschusses am Permanentmagneten separiert und in Wasser resuspendiert werden.

2. Verfahren zur Herstellung magnetischer Nanopartikel mit den Eigenschaften, dass sie a) 50 oder mehr Masseprozent Metall enthalten, b) hydrodynamische Durchmesser unterhalb von 200 nm aufweisen und c) bei Magnetfeldstärken unter 50 Oe in Suspension eine höhere Magnetisierung als das eingesetzte Metalloxid aufweisen, **gekennzeichnet dadurch, dass**
- eine wässrige Eisenoxidsuspension erwärmt und bei mindestens 500 bar ultrahomogenisiert wird,
- ein in Wasser gelöstes Polymer erwärmt und zur ultrahomogenisierten Eisenoxidsuspension gegeben wird,
- die Polymer-Eisenoxid-Suspension durch Zugabe einer Lauge neutralisiert wird und
- nach dem Abkühlen auf Raumtemperatur die erhaltenen magnetischen Nanopartikel zur Abtrennung des Polymerüberschusses mittels Hochgradientenmagnetfeld in Wasser gewaschen werden.

3. Verfahren zur Herstellung magnetischer Nanopartikel nach Anspruch 1 oder 2 **gekennzeichnet dadurch, dass** die Komponente Eisenoxid in situ aus entsprechenden Metallsalzen oder -hydroxiden erzeugt wird.

4. Verfahren zur Herstellung magnetischer Nanopartikel nach Anspruch 3 **gekennzeichnet dadurch, dass** es sich bei dem Trägermedium um eine Lösung von Ammoniak in Wasser handelt.

## Claims

1. A method for producing magnetic nanoparticles having the properties to
a) contain 50 percent by weight or more of metal;
b) comprise hydrodynamic diameters below 200 nm; and
c) have a higher magnetization in suspension at magnetic field strengths below 50 Oe than the used metal oxide;
**characterized in that**
- an aqueous iron oxide suspension is heated and ultra-homogenized at at least 500 bar;
- a polymer being dissolved in water is heated and added to said ultra-homogenized iron oxide suspension;
- said suspension of polymer and iron oxide is ultra-homogenized by further heating and at a pressure of 500 bar or above; and
- after cooling down to room temperature, said obtained magnetic nanoparticles are separated on a permanent magnet for the separation of excess polymer, and are resuspended in water.

2. A method for producing magnetic nanoparticles having the properties to
a) contain 50 percent by weight or more of metal;
b) comprise hydrodynamic diameters below 200 nm; and
c) have a higher magnetization in suspension at magnetic field strengths below 50 Oe than the used metal oxide;
**characterized in that**
- an aqueous iron oxide suspension is heated and ultra-homogenized at at least 500 bar;
- a polymer being dissolved in water is heated and added to said ultra-homogenized iron oxide suspension;
- said suspension of polymer and iron oxide is neutralized by adding an alkaline solution; and
- after cooling down to room temperature, said obtained magnetic nanoparticles are washed in water for the separation of excess polymer by means of a high gradient magnetic field.

3. A method for producing magnetic nanoparticles as claimed in claim 1 or 2,
**characterized in that**
the iron oxide component is produced in situ from respective metal salts or metal hydroxides.

4. A method for producing magnetic nanoparticles as claimed in claim 3,
**characterized in that**
the carrier medium is a solution of ammonia in water.

## Revendications

1. Procédé de fabrication de nanoparticules magnétiques présentant les propriétés selon lesquelles
a) elles comportent 50 pour cent en masse, ou plus, de métal,
b) elles présentent des diamètres hydrodynamiques inférieurs à 200 nm et
c) elles présentent, dans le cas d'intensité de champ magnétique inférieure à 50 Oe en suspension, une magnétisation plus élevée que l'oxyde métallique utilisé, **caractérisé en ce que**
- une suspension aqueuse d'oxyde de fer est chauffée et ultrahomogénéisée à au moins 500 bar,
- un polymère dissous dans de l'eau est chauffé et ajouté à la suspension d'oxyde de fer ultrahomogénéisée,
- la suspension de polymère et d'oxyde de fer est ultrahomogénéisée sous réchauffement poursuivi et sous une pression de 500 bar ou plus, et
- après refroidissement à température ambiante, les nanoparticules magnétiques obtenues sont tamisées pour séparer le surplus de polymères sur l'aimant permanent et sont resuspendues dans de l'eau.

2. Procédé de fabrication de nanoparticules magnétiques présentant les propriétés selon lesquelles a) elles comportent 50 pour cent en masse, ou plus, de métal, b) elles présentent des diamètres hydrodynamiques inférieurs à 200 nm et c) elles présentent, dans le cas d'intensité de champ magnétique inférieure à 50 Oe en suspension, une magnétisation plus élevée que l'oxyde métallique utilisé, **caractérisé en ce que**
- une suspension aqueuse d'oxyde de fer est chauffée et ultrahomogénéisée à au moins 500 bar,
- un polymère dissous dans l'eau est chauffé et ajouté à la suspension d'oxyde de fer ultrahomogénéisée,
- la suspension de polymère et d'oxyde de fer est neutralisée par addition d'une solution alcaline et
- après refroidissement à température ambiante, les nanoparticules magnétiques obtenues sont lavées dans de l'eau pour séparer le surplus de polymères au moyen d'un champ magnétique à gradient élevé.

3. Procédé de fabrication de nanoparticules magnétiques selon la revendication 1 ou 2, **caractérisé en ce que** le composant d'oxyde de fer est produit in situ à partir de sels métalliques ou d'hydroxydes métalliques correspondants.

4. Procédé de fabrication de nanoparticules magnétiques selon la revendication 3, **caractérisé en ce que** le milieu porteur est une solution d'ammoniaque dans de l'eau.
